# EUROPEAN PATENT APPLICATION

(11) **EP 1 743 521 A1**
(43) Date of publication of application: **17.01.2007**
(21) Application number: 05739027.0
(22) Date of filing: 28.04.2005
(51) Int. Cl.: A01K 67/00, G01N 33/15

(54) **METHOD OF CONSTRUCTING ANIMAL MODEL SUFFERING FROM LEFT VENTRICULAR DIASTOLIC DISORDER FOR EXAMINING HEART FAILURE AND METHOD OF EXAMINING REMEDY FOR HEART FAILURE CAUSED BY LEFT VENTRICULAR DIASTOLIC FAILURE WITH THE USE OF THE ANIMAL MODEL**

(30) Priority: 28.04.2004 JP 2004134900
(71) Applicant: AETAS PHARMA CO., LTD., Tokyo 103-0022 (JP)
(72) Inventor: KANEKO, Noboru, Oyama-shi, Tochigi 323-0022 (JP)
(74) Representative: Catherine, Alain
(86) International application number: PCT/JP2005/008557
(87) International publication number: WO 2005/104833

(57) **Abstract**

An animal model is provided which can be used for testing efficacy of a drug on left ventricular myocardial diastolic dysfunction, for example heart failure due to left ventricular diastolic failure.

The present invention resides in a method for preparing an animal model with left ventricular diastolic dysfunction for testing a therapeutic agent for heart failure, the method comprising: intravenously injecting an aqueous solution of a water-soluble calcium salt into an anesthetized experimental small animal; and intravenously injecting an aqueous catecholamine solution into the animal, while continuing the intravenous injection of the aqueous solution of the water-soluble calcium salt, to raise left ventricular end-diastolic pressure of the heart of the animal to a level higher than normal left ventricular end-diastolic pressure, and it also resides in a method for preparing an animal model with left ventricular diastolic dysfunction for testing a therapeutic agent for heart failure due to left ventricular diastolic failure, the method comprising: intravenously injecting an aqueous norepinepfrine solution into the animal model prepared by the former method; measuring left ventricular end-diastolic pressure; and comparing the measured left ventricular end-diastolic pressure with normal left ventricular end-diastolic pressure of before the injection of the aqueous norepinephrine solution.

## Description

### TECHNICAL FIELD

The present invention relates to a method for preparing an animal model with left ventricular diastolic dysfunction for testing a therapeutic agent for heart failure. In particular, it relates to a method for preparing an animal model with left ventricular diastolic dysfunction for testing a therapeutic agent for heart failure that is used for testing a therapeutic agent for heart failure due to left ventricular diastolic failure. The present invention also relates to a method for testing a therapeutic agent for heart failure due to left ventricular diastolic dysfunction. In particular, it relates to a method for testing a therapeutic agent for heart failure due to left ventricular diastolic dysfunction which uses the prepared animal model with left ventricular diastolic dysfunction for testing a therapeutic agent for heart failure.

### BACKGROUND ART

Heretofore, heart failure has been believed to occur due to myocardial systolic failure. Therefore, as a therapeutic agent for heart failure, drugs to enhance myocardial contraction have been used. Recently, however, it was found that in heart failure cases, there are many patients who developed heart failure although whose left ventricular systolic functions remains, and number of such patients amounts to as many as 40% of total heart failure patients in the cases. It has further revealed that in such cases, the patients do not necessarily have good prognoses. In such heart failure patients, no left ventricular dilatation is observed, and thus left ventricular diastolic failure due to left ventricular diastolic dysfunction is recognized to be cause of heart failure, which is called diastolic failure. Since such heart failure due to diastolic failure is different from heart failure due to systolic failure in mechanism of heart failure development, a sharp distinction is made between heart failure due to systolic failure and heart failure due to diastolic failure. Accordingly, methods for treating heart failure due to systolic failure and methods for treating heart failure due to diastolic failure are inevitably different. For treatment of left ventricular diastolic failure, use of a therapeutic agent in an acute exacerbation phase and use of a therapeutic agent in a chronic phase have been proposed. However, both of them are insufficient, and no drug to ameliorate left ventricular myocardial relaxation, i.e., drug to improve left ventricular diastolic failure as a specific medicine, has been found.

In general, tests to determine pharmacological activity of a novel drug or to determine unknown pharmacological activity of a known drug are carried out using experimental small animals as subjects to which the drug is administered. It is, therefore, most desirable to carry out, for example, tests to determine pharmacological activity of a drug for heart failure due to left ventricular diastolic failure on animal models with left ventricular diastolic failure. For this purpose, animal models with left ventricular diastolic failure are needed. Heretofore, however, animal models with left ventricular diastolic failure have been hard-to-find, and because of the limited availability, no specifically efficacious therapeutic agents for heart failure due to left ventricular diastolic failure have been found yet. Accordingly, it is recognized that cure of patients with heart failure due to left ventricular diastolic failure is difficult.

It is an object of the present invention to provide an animal model for testing pharmacological activity of a drug to seek a specifically efficacious therapeutic agent for left ventricular myocardial relaxation failure, for example, heart failure due to left ventricular diastolic failure which has been said to account for 40% of heart failure.

### DISCLOSURE OF INVENTION

The present invention provides an animal model which can be used for testing efficacy of a drug on heart failure due to myocardial diastolic failure, for example, heart failure due to left ventricular diastolic failure.

In other words, the present invention resides in a method for preparing an animal model with left ventricular diastolic dysfunction for testing a therapeutic agent for heart failure, the method comprising:
intravenously injecting an aqueous solution of a water-soluble calcium salt into an anesthetized experimental small animal; and
intravenously injecting an aqueous catecholamine solution into the animal, while continuing the intravenous injection of the aqueous solution of the water-soluble calcium salt, to raise left ventricular end-diastolic pressure of the heart of the animal to a level higher than normal left ventricular end-diastolic pressure. Further, the present invention resides in a method for preparing an animal model with left ventricular diastolic dysfunction for testing a therapeutic agent for heart failure, the method comprising:
intravenously injecting an aqueous solution of a water-soluble calcium salt into an experimental small animal anesthetized with urethane and α-chloralose; and
intravenously injecting an aqueous catecholamine solution into the animal, while continuing the intravenous injection of the aqueous solution of the water-soluble calcium salt, to raise left ventricular end-diastolic pressure of the heart of the animal to a level higher than normal left ventricular end-diastolic pressure. Moreover, the present invention resides in a method for testing a therapeutic agent for heart failure due to left ventricular diastolic failure by means of an animal model with left ventricular diastolic dysfunction for heart failure test, the method comprising:
intravenously injecting an aqueous solution of a water-soluble calcium salt into an anesthetized experimental small animal;
intravenously injecting an aqueous catecholamine solution into the animal, while continuing the intravenous injection of the aqueous solution of the water-soluble calcium salt, to raise left ventricular end-diastolic pressure of the heart of the animal to a level higher than normal left ventricular end-diastolic pressure;
intravenously injecting a test drug into the animal of which left ventricular end-diastolic pressure has been raised to the level;
measuring left ventricular end-diastolic pressure; and
comparing the measured left ventricular end-diastolic pressure with normal left ventricular end-diastolic pressure before the injection of the aqueous catecholamine solution. Furthermore, the present invention resides in a method for testing a therapeutic agent for heart failure due to left ventricular diastolic failure by means of an animal model with left ventricular diastolic dysfunction for heart failure test, the method comprising:
intravenously injecting an aqueous solution of a water-soluble calcium salt into an experimental small animal anesthetized with urethane and α-chloralose;
intravenously injecting an aqueous catecholamine solution into the animal, while continuing the intravenous injection of the aqueous solution of the water-soluble calcium salt, to raise left ventricular end-diastolic pressure of the heart of the animal to a level higher than normal left ventricular end-diastolic pressure;
intravenously injecting a test drug into the animal of which left ventricular end-diastolic pressure has been raised to the level;
measuring left ventricular end-diastolic pressure; and
comparing the measured left ventricular end-diastolic pressure with normal left ventricular end-diastolic pressure before the injection of the aqueous catecholamine solution.

In the method for preparing an animal model for testing a therapeutic agent for heart failure due to myocardial relaxation dysfunction, injection rate of the aqueous solution of the water-soluble calcium salt may be 4 mg/kg/min to 16 mg/kg/min in terms of calcium chloride, and injection rate of the aqueous catecholamine solution may be 1 µg/kg/min to 120 µg/kg/min in terms of norepinephrine. In the method for testing a therapeutic agent for left ventricular diastolic failure by means of an animal model for heart failure test with heart failure due to myocardial relaxation dysfunction, injection rate of the aqueous solution of the water-soluble calcium salt may be 4 mg/kg/min to 16 mg/kg/min in terms of calcium chloride, and injection rate of the aqueous catecholamine solution may be 1 µg/kg/min to 120 µg/kg/min in terms of norepinephrine. Further, in the drug test according to the present invention, the measurement of left ventricular end-diastolic pressure may be performed 5 minutes after the intravenous injection of the aqueous catecholamine solution.

The present invention is capable of easily preparing an animal model with left ventricular diastolic dysfunction for testing a therapeutic agent for heart failure, by intravenously injecting an aqueous solution of a water-soluble calcium salt such as an aqueous solution of calcium chloride into an anesthetized experimental small animal, and intravenously injecting an aqueous catecholamine solution into the animal, while continuing the intravenous injection of the aqueous solution of the water-soluble calcium salt such as an aqueous solution of calcium chloride, to raise left ventricular end-diastolic pressure of the heart of the animal to a level higher than normal left ventricular end-diastolic pressure. The use of the thus prepared animal model for testing a therapeutic agent for heart failure due to impaired myocardial relaxation makes it easy to test in vivo whether or not the analyte drug has pharmaceutical activity as a therapeutic agent for heart failure due to left ventricular diastolic failure. As described above, according to the present invention, it is facilitated to find drugs having pharmaceutical activities as therapeutic agents for heart failure due to left ventricular diastolic failure. This contributes to promotion of providing novel therapeutic drugs for heart failure due to left ventricular diastolic failure.

### BEST MODE FOR CARRYING OUT THE INVENTION

In the present invention, an aqueous solution of a water-soluble calcium salt such as an aqueous solution of calcium chloride is intravenously injected into an anesthetized experimental small animal, and an aqueous catecholamine solution, for example, an aqueous norepinephrine solution, i.e., an aqueous noradrenaline solution is intravenously injected into the animal, while continuing the intravenous injection of the aqueous solution of calcium chloride, to raise left ventricular end-diastolic pressure of the heart of the animal to a level higher than normal left ventricular end-diastolic pressure, thereby easily preparing an animal model with left ventricular diastolic dysfunction for testing a therapeutic agent for heart failure. In the present invention, in the preparation of the test animal, the injection rate of the aqueous solution of a water-soluble calcium salt is preferably 4 mg/kg/min to 16 mg/kg/min in terms of calcium chloride (in terms of calcium, 1.45 mg/kg/min to 5.78 mg/kg/min). The injection rate of the aqueous catecholamine solution is preferably 1 µg/kg/min to 120 µg/kg/min in terms of norepinephrine. More preferably, however, the injection rate in terms of norepinephrine is 20 µg/kg/min to 60 µg/kg/min because such an intended test animal model can easily be prepared. Further preferably, the injection rate in terms of norepinephrine is 35 µg/kg/min to 45 µg/kg/min because such a test animal model can be prepared more easily and reliably. If the injection rate in terms of norepinephrine is higher than 120 µg/kg/min, sharp increase in blood pressure is caused, sometimes leading to death of the experimental small animal. Undesirably, this gives rise to increase in amount of a drug to suppress sharp increase in blood pressure and thus evaluation of effect of the test drug is difficult. On the other hand, if the injection rate in terms of norepinephrine is lower than 1 µg/kg/min, yield of the test animal models becomes undesirably low. In the present invention, calcium chloride acts in cooperation with norepinephrine which is injected together therewith. Accordingly, if the injection rate in terms of calcium chloride is higher than 16 mg/kg/min, arrhythmia is likely to be caused, undesirably sometimes leading to death of the experimental small animal. If the injection rate in terms of calcium chloride is lower than 4 mg/kg/min, yield of the test animal models becomes undesirably low.

The aqueous solution of calcium chloride may be prepared by dissolving calcium chloride in a glucose solution or physiological saline. It is, however, preferred to prepare the aqueous calcium chloride solution by dissolving calcium chloride in a glucose solution. The aqueous norepinephrine solution may be prepared by dissolving norepinephrine in distilled water. The intravenous injection of the aqueous calcium chloride solution and the intravenous injection of the norepinephrine solution are carried out preferably in different veins or at different points of the same vein. In the present invention, experimental small animals are used to obtain the test animal models. As the experimental small animals, for example, rats, guinea pigs, rabbits, dogs, and miniature pigs may be used. However, the experimental small animals are not restricted to these small animals. The experimental small animal is preliminarily anesthetized with, for example, urethane and α-chloralose or the like in a usual manner.

### EXAMPLES

Examples of the present invention will be described hereinbelow. It should be, however, understood that the present invention is by no means limited by the following demonstrations or descriptions.

### EXAMPLE 1

In this Example, male Wistar rats of 8 weeks of age and 300g-330g in body weight were used. Anesthesia was effected by subperitoneally injecting 1000 mg/kg of urethane and 80 mg/kg of α-chloralose, and the rats were allowed to breath spontaneously. In this Example, calcium chloride was dissolved in a 5% glucose solution to prepare a calcium chloride solution, and 1 mg of norepinephrine was dissolved in 41 µl of distilled water to prepare an aqueous norepinephrine solution. In this Example, monohydrochloride of 4-[3-(4-benzylpiperidin-1-yl) propionyl]-7-methoxy-2,3,4,5-tetrahydro-1,4-benzothiazepine (hereinafter referred to as the above-mentioned compound) was used as a test drug for treatment of heart failure due to left ventricular diastolic failure. 100mg of the above-mentioned compound was dissolved in 1ml of dimethyl sulfoxide (DMSO) as a solvent to prepare a solution of the above-mentioned compound, and the solution of the above-mentioned compound was stored at a temperature of 4°C.

In this Example, a catheter for continuous injection of the aqueous calcium chloride solution or an aqueous norepinephrine solution containing calcium chloride was inserted into the right external jugular vein of each of the rats, and a catheter for continuous injection of the solution of the test drug was inserted into the right femoral vein, and a microchip catheter (model SPC-320 manufactured by Millar Co.) was inserted into the left ventricle through the right common carotid artery.

Electrocardiograms were taken in lead I, and the electrocardiograms and left ventricular pressures were in parallel recorded in a personal computer via an A/D converter. Pressures corresponding to electrocardiographic R-waves were measured as left ventricular end-diastolic pressures with respect to 20 pulses, and the average value thereof was taken and regarded as a left ventricular end-diastolic pressure at the measuring time. Blood pressures, pulses and electrocardiograms of the rats were monitored for 15 minutes, and when these became stable, the solution of calcium chloride in 5% glucose solution of which calcium chloride concentration was adjusted according to the body weight of each of the rats was injected from the right external jugular vein for 20 minutes at a rate of 16.6µl/min (calcium chloride: 9.0 mg/kg/min) as a pretreatment.

Immediately thereafter, injection of the aqueous norepinephrine solution containing calcium chloride from the right external jugular vein was initiated wherein the manner of injection and dose of calcium chloride were not changed from those in the above-mentioned aqueous calcium chloride solution and wherein the norepinephrine solution was injected at a rate of 40 µg/kg/min in terms of norepinephrine. After the initiation, the injection of calcium chloride and the injection of norepinephrine by injecting the aqueous norepinephrine solution containing calcium chloride was continued. 5 minutes after the initiation of the injection (intravenous injection) of the aqueous solution of calcium chloride and norepinephrine from the right external jugular vein, 0.2ml of physiological saline as a control material was injected into a first rat of 300mg in body weight as a subject from the right femoral vein over a period of 30 seconds, while continuing the injection of calcium chloride and the injection of norepinephrine by the injection of the aqueous norepinephrine solution containing calcium chloride from the right external jugular vein, to thereby obtain Subject 1. Into a second rat of 310g in body weight as a subject, 5 minutes after the initiation of the injection (intravenous injection) of the aqueous norepinephrine solution containing calcium chloride from the right external jugular vein, 0.2ml of a 1 % aqueous solution of dimethyl sulfoxide (DMSO) which is a solvent as a control material was injected from the right femoral vein over a period of 30 seconds, while continuing the injection of calcium chloride and the injection of norepinephrine by the injection of the aqueous norepinephrine solution containing calcium chloride from the right external jugular vein, to thereby obtain Subject 2. Into a third rat of 310g in body weight as a subject, 5 minutes after the initiation of the injection (intravenous injection) of the aqueous norepinephrine solution containing calcium chloride from the right external jugular vein, 0.2ml of a 1 % DMSO aqueous solution containing 0.3mg/kg of the above-mentioned compound as a test drug of which pharmacological activity on left ventricular diastolic heart failure was to be examined was injected from the right femoral vein over a period of 30 seconds, while continuing the injection of calcium chloride and the injection of norepinephrine by the injection of the aqueous norepinephrine solution containing the calcium chloride solution from the right external jugular vein, to thereby obtain Subject 3. Into a fourth rat of 330g in body weight as a subject, 5 minutes after the initiation of the injection (intravenous injection) of the aqueous norepinephrine solution containing calcium chloride from the right external jugular vein, 0.2ml of a 1% DMSO aqueous solution containing 0.3mg/kg of the above-mentioned compound as a test drug of which pharmacological activity on left ventricular diastolic heart failure was to be examined was injected from the right femoral vein over a period of 30 seconds, while continuing the injection of calcium chloride and the injection of norepinephrine by the injection of the aqueous norepinephrine solution containing the calcium chloride solution from the right external jugular vein, to thereby obtain Subject 4. In this Example, with respect to each of Subjects 1 to 4, even after the injection of the control material or test drug over a period of 30 minutes, the injection of calcium chloride and norepinephrine from the right external jugular vein was continued. In this Example, with respect to each of the rats of Subjects 1 and 2, after the injection of the control material over a period of 30 seconds, a diluted control material solution having a concentration of one tenth of that of the injected control material solution was additionally injected at a rate of 10µl/min until 15 minutes after the initiation of the injection of the control material. In this Example, with respect to Subject 3, after the injection of 0.2ml of the 1% DMSO solution containing 0.3 mg/kg of the above-mentioned compound as a test drug over a period of 30 seconds, the 1% DMSO solution containing the above-mentioned compound was additionally injected at a rate of 0.02 mg/kg/min in terms of the above-mentioned compound until 15 minutes after the initiation of the injection of the test drug. With respect to Subject 4, however, only the injection of the above-mentioned test drug over a period of 30 seconds was carried out, and no additional injection of the test drug was carried out. With respect to each of the rats, pressures corresponding to electrocardiographic R-waves were measured with respect to 20 pulses, and the average value thereof was taken every minute and regarded as a left ventricular end-diastolic pressure. 15 minutes after the initiation of the injection of the control material or test drug, the test for assaying the test drug was finished. The results in this test at every 5 minutes are shown in the following Table 1.

**Table 1**

| Elapsed time | Left ventricular end-diastolic pressure (unit: mmHg) | | | |
|---|---|---|---|---|
| | Subject 1 (physiological saline) | Subject 2 (solvent) | Subject 3 (above-mentioned compound) | Subject 4 (above-mentioned compound) |
| 5 min before (5 min before the initiation of administration of CaCl₂) | 7.7 | 7.6 | 7.5 | 8.4 |
| 0 min (the initiation of administration of CaCl₂) | 7.5 | 7.6 | 7.8 | 8.6 |
| 5 min after(5 min after the initiation of administration of CaCl₂) | 8.4 | 8.2 | 7.9 | 8.8 |
| 10 min after (10 min after the initiation of administration of CaCl₂) | 7.5 | 7.8 | 8.6 | 9.2 |
| 15 min after (15 min after the initiation of administration of CaCl₂) | 8.5 | 8.4 | 8.5 | 9.0 |
| 20 min after (immediately before the initiation of intravenous injection of norepinephrine) | 8.6 | 8.6 | 8.6 | 9.3 |
| 25 min after (immediately before the initiation of intravenous injection of the control material or test drug) | 7.8 | 8.8 | 8.9 | 9.6 |
| 30 min after (5 min after the initiation of intravenous injection of the control material or test drug) | 11.9 | 10.1 | 10.6 | 13.8 |
| 35 min after (10 min after the initiation of intravenous injection of the control material or test drug) | 30.4 | 37.5 | 12.5 | 16.4 |
| 40 min after (15 min after the initiation of intravenous injection of the control material or test drug) | 47.3 | 49.4 | 11.8 | 15.3 |

| | | | | |
|---|---|---|---|---|
| (Note 1) The "before" of "5 min before" means "before the initiation of administration of calcium chloride", and the "after" of "5 min after", ------, or "40 min after" means "after the initiation of administration of calcium chloride". (Note 2) The materials shown in parentheses in the columns of Subjects 1 to 4 are control materials or test drug. | | | | |

This Example was performed at room temperature of 20 - 25°C. In this Example, in the period from after the administration of calcium chloride to immediately before the initiation of the intravenous injection of norepinephrine, the left ventricular end-diastolic pressures were 7.7-8.5 mmHg in Subject 1, 7.6-8.6 mmHg in Subject 2, 7.5-8.6 mmHg in Subject 3, and 8.4-9.3 mmHg in Subject 4. In the period from after the administration of calcium chloride to immediately before the intravenous injection of norepinephrine, no substantial changes were observed in the left ventricular end-diastolic pressures in Subjects 1 to 4. However, the left ventricular end-diastolic pressures in the period from 10 minutes after the initiation of the intravenous injection of the norepinephrine solution increased to 11.9-47.3 mmHg in Subject 1, and also increased to 10.1-49.4 mmHg in Subject 2. In both Subjects 1 and 2, left ventricular diastolic failure developed and continued.

In contrast thereto, in Subject 3 as the case for testing the solution of the above-mentioned compound as a test drug, the left ventricular end-diastolic pressure increased to 10.1 mmHg 10 minutes after the initiation of the intravenous injection of the norepinephrine solution, so that onset of left ventricular diastolic failure was observed. In Subject 3, however, although the left ventricular end-diastolic pressure increased to 12.5 mmHg 10 minutes after the initiation of the intravenous injection of the solution of the above-mentioned compound as a test drug (15 minutes after the initiation of the intravenous injection of the norepinephrine solution), the left ventricular end-diastolic pressure decreased to 11.8 mmHg 15 minutes after the initiation of the intravenous injection of the solution of the above-mentioned compound as a test drug (20 minutes after the initiation of the intravenous injection of the norepinephrine solution). This left ventricular end-diastolic pressure of Subject 3 is lower than 1/2 of those of Subjects 1 and 2 at the corresponding times. This shows that lowering of left ventricular end-diastolic pressure was realized and that by the intravenous injection of the above-mentioned compound as a test drug, left ventricular diastolic failure was prevented from occurring.

Also in Subject 4 as the case for testing the solution of the above-mentioned compound as a test drug, although the left ventricular end-diastolic pressure increased to 16.4 mmHg 10 minutes after the initiation of the intravenous injection of the solution of the above-mentioned compound as a test drug (15 minutes after the initiation of the intravenous injection of the norepinephrine solution), the left ventricular end-diastolic pressure decreased to 15.3 mmHg 15 minutes after the initiation of the intravenous injection of the solution of the above-mentioned compound as a test drug (20 minutes after the initiation of the intravenous injection of the norepinephrine solution). This left ventricular end-diastolic pressure of Subject 4 is lower than 1/2 of those of Subjects 1 and 2 at the corresponding times. This shows that lowering of left ventricular end-diastolic pressure was realized and that by the intravenous injection of the above-mentioned compound as a test drug, left ventricular diastolic failure was prevented from occurring.

It is understood from the results in Subjects 3 and 4 that by carrying out the additional intravenous injection of the test drug, the better results of the test on the test drug were obtained, and this renders evaluation of the test results easier. Further, it is evident from the results in Subjects 1-4 that the above-mentioned compound as a test drug is effective as a therapeutic agent or prophylactic agent for left ventricular diastolic failure.

### EXAMPLE 2

Test on left ventricular diastolic failure from the viewpoint of left ventricular myocardial diastolic wall motion velocity by means of a tissue Doppler method.

In this Example, male Wistar rats of 9 weeks of age and 310g and 320g in body weight were used. Anesthesia was effected by subperitoneally injecting 1000mg/kg of urethane and 80mg/kg of α-chloralose, and the rats were allowed to breath spontaneously. In this Example, monohydrochloride of 4-[3-(4-benzylpiperidin-1-yl)propionyl]-7-methoxy-2,3,4,5-tetrahydro-1,4-benzothiazepine (hereinafter referred to as the above-mentioned compound) was used as a test drug for treatment of heart failure due to left ventricular diastolic failure as in Example 1. As a control material, a 1 % aqueous solution of dimethyl sulfoxide (DMSO) was used as in Example 1.

In this Example, 100mg of the above-mentioned compound was dissolved in 1 ml of dimethyl sulfoxide (DMSO) to prepare a solution of the above-mentioned compound in DMSO, and this solution was stored at a temperature of 4°C. 1 mg of norepinephrine was dissolved in 41 µl of distilled water to prepare a norepinephrine solution.

This Example was performed at room temperature of 20-25°C, as in Example 1. Also in this Example, a catheter for continuous injection of an aqueous calcium chloride solution or an aqueous norepinephrine solution containing calcium chloride was inserted into the right external jugular vein of each of the rats, and a microchip catheter (model SPC-320 manufactured by Millar Co.) was inserted into the aorta through the right common carotid artery, as in Example 1.

Electrocardiograms were taken in lead I, and blood pressures, pulses and electrocardiograms of the rats were monitored for 15 minutes, and when these became stable, an aqueous solution of calcium chloride prepared by dissolving calcium chloride in a 5% aqueous glucose solution was injected from the right external jugular vein for 25 minutes at a rate of 16.6 µl/min (9.0 mg/kg/min in terms of calcium chloride) as a pretreatment.

Immediately thereafter, injection of the norepinephrine solution from the right external jugular vein at a rate of 40 µg/kg/min in terms of norepinephrine was initiated while injecting the aqueous calcium chloride solution with the injection manner and dose unchanged. Into a first rat of 310mg in body weight as a subject, 5 minutes after the initiation of the injection (intravenous injection) of the aqueous norepinephrine solution containing calcium chloride from the right external jugular vein, 0.2ml of a 1 % aqueous solution of DMSO which is a solvent as a control material only was injected from the right femoral vein over a period of 30 seconds, while continuing the injection of calcium chloride and the injection of norepinephrine by the injection of the aqueous norepinephrine solution containing the calcium chloride solution from the right external jugular vein, to thereby obtain Subject 5. Into a second rat of 320g in body weight as a subject, 5 minutes after the initiation of the injection (intravenous injection) of the aqueous norepinephrine solution containing calcium chloride from the right external jugular vein as in the case of Subject 5, 0.2ml of a 1 % aqueous DMSO solution containing 0.3 mg/kg of the above-mentioned compound as a test drug was injected from the right femoral vein over a period of 30 seconds as in the case of Subject 5, while continuing the injection of calcium chloride and the injection of norepinephrine by the injection of the aqueous norepinephrine solution containing the calcium chloride solution from the right external jugular vein, to thereby obtain Subject 6. Into Subject 6, the above-mentioned compound as a test drug was thereafter additionally injected at a rate of 0.02 g/kg/min for 20 minutes. With respect to each of the rats, diastolic wall motions of left ventricular wall were examined using ultrasonic diagnostic equipment. As the ultrasonic diagnostic equipment, one manufactured by Toshiba Corp. (Toshiba Powervision SSA-380APSK-70LT model) was used, and the examination was performed with ultrasonic wave of 10MHz. The examination with ultrasonic wave was performed in such a manner that with respect to each of the rats, a precordial region was first shaved, and an echo probe was applied to the apex of heart to create an image of an apical left ventricular long axis layer, and sample volume of pulse Doppler was located on a base portion of the posterior leaflet of mitral valve to measure Ea wave of diastolic wall motion velocities of left ventricular wall. Ea wave (m/sec) of wall motion velocity before the administration of norepinephrine was determined as 1.00, and ratios of Ea wave (m/sec) of wall motion velocities after the administration of the control material or test drug thereto were obtained. The results are shown in the following Table 2.

**Table 2**

| Elapsed time | Ratio between left ventricular diastolic wall motion velocities | |
|---|---|---|
| | Subject 5 (solvent) | Subject 6 (above-mentioned compound) |
| 5 min before (immediately before the initiation of intravenous injection of norepinephrine) | 1.00 | 1.00 |
| 0 min (immediately before of min after the initiation of intravenous injection of the control material or test drug) | 1.00 | 1.00 |
| 5 min (5 min after the initiation of intravenous injection of the control material or test drug) | 0.85 | 1.02 |
| 10 min (10 min after the initiation of intravenous injection of the control material of test drug) | 0.75 | 1.04 |
| 15 min (15 min after the initiation of intravenous injection of the control material or test drug) | 0.70 | 1.00 |
| 20 min (20 min after the initiation of intravenous injection of the control material or test drug) | 0.60 | 1.00 |
| 25 min (25 min after the initiation of intravenous injection of the control material or test drug) | 0.55 | 0.94 |
| 30 min (30 min after the initiation of intravenous injection of the control material or test drug) | 0.51 | 0.95 |

| | | |
|---|---|---|
| (Note 1) The "5 min before" means 5 minutes before the initiation of injection of the control material or test drug. (Note 2) The "0 min", ---- or "30 min" means elapsed time of "0 min",--- or "30 min" after the initiation of intravenous injection of the control material or test drug. | | |

In this Example, 5 minutes after the initiation of the injection of norepinephrine, the velocity ratio of Ea wave of wall motion velocity of left ventricular wall to that at a time around the administration of norepinephrine in Subject 5 decreased to 0.85. This shows lowering of left ventricular diastolic function. In Subject 5, the ratio further decreased. 30 minutes after the initiation of the injection of norepinephrine, the velocity ratio of Ea wave of wall motion velocity of the left ventricular wall in Subject 5 decreased to 0.51, showing further lowering of left ventricular diastolic function. This shows that in Subject 5, diastolic dysfunction occurred in the left ventricular cardiac muscle to develop left ventricular diastolic failure, and 30 minutes after the initiation of the injection of norepinephrine, left ventricular diastolic failure further progressed. In contrast thereto, in the case of Subject 6 to which the above-mentioned compound was administered, the velocity ratio of Ea wave of wall motion velocity of the left ventricular wall to that at a time around the administration of norepinephrine was 1.00-1.04 until 20 minutes after the initiation of the initiation of norepinephrine, and this shows that no substantial changes were observed in left ventricular diastolic function. Even 30 minutes after the initiation of the initiation of norepinephrine, the velocity ratio of Ea wave of wall motion velocity of the left ventricular wall to that at a time around the administration of norepinephrine was 0.95, and this shows that no substantial change was observed in the velocity ratio of Ea wave of wall motion velocity of left ventricular wall and thus no substantial change was observed in left ventricular diastolic function. It is, therefore, shown that in the case where the above-mentioned compound was administered, even after the initiation of the injection of norepinephrine, left ventricular diastolic failure was cured or prevented by virtue of the administration of the above-mentioned compound.

It is shown from the results of Examples 1 and 2 that the administration of the above-mentioned compound can cure diastolic dysfunction of left ventricular cardiac muscle or prevent diastolic dysfunction of left ventricular cardiac muscle from occurring, and that the above-mentioned compound is capable of being used as a therapeutic agent or prophylactic agent for heart failure due to left ventricular diastolic failure.

### INDUSTRIAL APPLICABILITY

The present invention facilitates testing in vivo whether or not a drug has pharmaceutical activity as a therapeutic agent for heart failure due to left ventricular diastolic failure, and thus the present invention is helpful to developments of therapeutic agents for heart failure due to left ventricular diastolic failure. Therefore, cure of heart failure due to left ventricular diastolic failure which has been recognized to be hard to cure is realized, and the present invention contributes to decrease in mortality by heart failure due to left ventricular diastolic failure, thereby greatly contributing to society.

## Claims

1. A method for preparing an animal model with left ventricular diastolic dysfunction for testing a therapeutic agent for heart failure, said method comprising:
intravenously injecting an aqueous solution of a water-soluble calcium salt into an anesthetized experimental small animal; and
intravenously injecting an aqueous catecholamine solution into said animal, while continuing the intravenous injection of the aqueous solution of the water-soluble calcium salt, to raise left ventricular end-diastolic pressure of the heart of said animal to a level higher than normal left ventricular end-diastolic pressure.

2. A method for preparing an animal model with left ventricular diastolic dysfunction for testing a therapeutic agent for heart failure, said method comprising:
intravenously injecting an aqueous solution of a water-soluble calcium salt into an experimental small animal anesthetized with urethane and α-chloralose; and
intravenously injecting an aqueous catecholamine solution into said animal, while continuing the intravenous injection of the aqueous solution of the water-soluble calcium salt, to raise left ventricular end-diastolic pressure of the heart of said animal to a level higher than normal left ventricular end-diastolic pressure.

3. The method for preparing an animal model with left ventricular diastolic dysfunction for testing a therapeutic agent for heart failure according to claim 1 or 2, wherein injection rate of the aqueous solution of the water-soluble calcium salt is 4 mg/kg/min to 16 mg/kg/min in terms of calcium chloride.

4. The method for preparing an animal model with left ventricular diastolic dysfunction for testing a therapeutic agent for heart failure according to claim 1 or 2, wherein injection rate of the aqueous catecholamine solution is 1 µg/kg/min to 120 µg/kg/min in terms of norepinephrine.

5. A method for testing a therapeutic agent for heart failure due to left ventricular diastolic failure by means of an animal model with left ventricular diastolic dysfunction for heart failure test, said method comprising:
intravenously injecting an aqueous solution of a water-soluble calcium salt into an anesthetized experimental small animal;
intravenously injecting an aqueous catecholamine solution into said animal, while continuing the intravenous injection of the aqueous solution of the water-soluble calcium salt, to raise left ventricular end-diastolic pressure of the heart of said animal to a level higher than normal left ventricular end-diastolic pressure;
intravenously injecting a test drug into said animal of which left ventricular end-diastolic pressure has been raised to the level;
measuring left ventricular end-diastolic pressure; and
comparing the measured left ventricular end-diastolic pressure with normal left ventricular end-diastolic pressure of before the injection of the aqueous catecholamine solution.

6. A method for testing a therapeutic agent for heart failure due to left ventricular diastolic failure by means of an animal model with left ventricular diastolic dysfunction for heart failure test, said method comprising:
intravenously injecting an aqueous solution of a water-soluble calcium salt into an experimental small animal anesthetized with urethane and α-chloralose;
intravenously injecting an aqueous catecholamine solution into said animal, while continuing the intravenous injection of the aqueous solution of the water-soluble calcium salt, to raise left ventricular end-diastolic pressure of the heart of said animal to a level higher than normal left ventricular end-diastolic pressure;
intravenously injecting a test drug into said animal of which left ventricular end-diastolic pressure has been raised to the level;
measuring left ventricular end-diastolic pressure; and
comparing the measured left ventricular end-diastolic pressure with normal left ventricular end-diastolic pressure of before the injection of the aqueous catecholamine solution.

7. The method according to claim 5 or 6 for testing a therapeutic agent for heart failure due to left ventricular diastolic failure by means of an animal model with left ventricular diastolic dysfunction for heart failure test, wherein the measurement of left ventricular end-diastolic pressure is performed 5 minutes after the intravenous injection of the aqueous catecholamine solution.

8. The method according to claim 5 or 6 for testing a therapeutic agent for heart failure due to left ventricular diastolic failure by means of an animal model with left ventricular diastolic dysfunction for heart failure test, wherein injection rate of the aqueous solution of the water-soluble calcium salt is 4 mg/kg/min to 16 mg/kg/min in terms of calcium chloride.

9. The method according to claim 5 or 6 for testing a therapeutic agent for heart failure due to left ventricular diastolic failure by means of an animal model with left ventricular diastolic dysfunction for heart failure test, wherein injection rate of the aqueous catecholamine solution is 1 µg/kg/min to 120 µg/kg/min in terms of norepinephrine.
